# EUROPÄISCHE PATENTSCHRIFT

(11) **EP 1 850 884 B1**
(45) Veröffentlichungstag und Bekanntmachung des Hinweises auf die Patenterteilung: **23.06.2010**
(21) Anmeldenummer: 06701479.5
(22) Anmeldetag: 26.01.2006
(51) Int. Cl.: A61L 9/00

(54) **LÖSUNGSMITTELFREI HERSTELLBARE DISPERSION ENTHALTEND EINEN 1:1- ODER 2:1-CYCLODEXTRIN-RIECHSTOFF-KOMPLEX**
DISPERSION OBTAINABLE WITHOUT SOLVENTS AND CONTAINING A 1:1 OR 2:1 CYCLODEXTRIN-ODORIFEROUS SUBSTANCE COMPLEX
DISPERSION REALISEE SANS SOLVANT ET CONTENANT UN COMPLEXE DE MATIERES ODORANTES ET DE CYCLODEXTRINE DANS UN RAPPORT DE 1:1 OU DE 2:1

(30) Priorität: 08.02.2005 DE 102005005633
(43) Veröffentlichungstag der Anmeldung: 07.11.2007
(73) Patentinhaber: Wacker Chemie AG, 81737 München (DE)
(72) Erfinder: REGIERT, Marlies, 80538 München (DE); KUPKA, Michaela, 84489 Burghausen (DE); ZEH, Harald, 84489 Burghausen (DE)
(74) Vertreter: Potten, Holger
(86) Internationale Anmeldenummer: PCT/EP2006/000694
(87) Internationale Veröffentlichungsnummer: WO 2006/084586

(56) Entgegenhaltungen:
- EP-A- 0 460 588
- WO-A-01/16264
- DE-A1- 10 101 294
- US-A- 4 356 115
- US-A- 4 722 815

## Beschreibung

Die Erfindung betrifft eine lösungsmittelfrei herstellbare Dispersion enthaltend einen 1:1- oder 2:1-Cyclodextrin-Riechstoff-Komplex.

Cyclodextrine sind cyclische Oligosaccharide, die aus 6, 7 oder 8 α(1-4)-verknüpften Anhydroglukoseeinheiten aufgebaut sind. Die durch enzymatische Stärkekonversion hergestellten α-, β- oder γ-Cyclodextrine unterscheiden sich im Durchmesser ihrer hydrophoben Kavität und eignen sich generell zum Einschluss zahlreicher lipophiler Substanzen. Die Komplexierung von Riechstoffen mittels Cyclodextrinen ist beispielsweise aus Proceedings of the first Internation Symposium on cyclodextrins, Budapest, Hungary, 30 September - 2 October, 1981, "Cyclodextrins in foods, cosmetics and toiletries", S. 469-480 bekannt.

Unter Riechstoffen sind im Sinne der vorliegenden Erfindung Stoffe od. Stoffgemische, welche durch Geruch wahrnehmbar sind, zu verstehen, wobei zwischen Duftstoffen mit angenehmer Wirkung u. Stinkstoffen mit Erzeugung unangenehmer Empfindung bis zu Ekel u. Erbrechen unterschieden werden kann. Auch Repellentien zählen zu den Riechstoffen mit Erzeugung unangenehmer Empfindung. Sie weisen eine insektenabweisende Wirkung auf. Viele der Mittel veranlassen durch ihren unangenehmen Geruch und/oder Geschmack, dass die Tiere sich von z. B. Nahrung od. bestimmten Plätzen fernhalten. Hierzu gehören Mittel zur Abschreckung von Insekten u. Spinnen, zu denen außer den bei Insektenabwehrmitteln genannten Stoffen (z. B. N,N-diethylmeta-toluamide (DEET)), auch Octansäurediethylamid, 2-(Octylthio)-ethanol, N-Benzylpiperidine u. 3-(N-Acetyl-N-butylamino)-propionsäureethylester zählen. Zu den Riechstoffen zählen auch alle Mittel, die zur Vertreibung von Nagetieren, Vögeln z. B. in Obstkulturen, Weinbergen usw. dienen, Wildverbissmittel zum Schutz der jungen Forstkulturen sowie Mittel, die Hunde u. Katzen abschrecken sollen, bestimmte Stellen zu verunreinigen (z. B. 2-Undecanon). Gerade im Bautenschutz von Altstadtfasaden sind Repellentien gegen Vögel im Besonderen gegen Wildtauben von Interesse. Folgende Riechstoffe weisen eine durch ihren Geruch abweisende Wirkung auf Vögel auf Dimethylanthranilate, Methylantranilat, Phenylethylantranilat, Dimethylbenzylcarbonylacetat, Methylphenyl acetate, orthoamino-Acetophenon, 2-amino-4,5-dimethyl-Acetophenone, Veratroylamine, Zimtaldehyd, Zimtsäureamide, Zimtsäure und Kombinationen davon.

Viele Riechstoffe weisen eine fungizide oder bakterizide Wirkung auf: Phenole wie Thymol, Eugenol, etherische Öle wie Thymian-, Nelkenblüten-, Teebaum-, Oreganum- und Lemongras-Öl.

Synthetische Riechstoffe (DEET, Amide, Imide, Alkohole, Phenole mit einem Siedpunkt von 100 - 300°C) wie auch natürliche Riechstoffe der Substanzgruppe etherische Öle, wie Bergamotte-, Birkenteer-, Campher-, Citronella-, Eukalyptus-, Muskatnuss-, Nelkenblüten-, Orangenblüten-, Pfefferminz-, Pyrethrum-, Thymian-, Zimtrinden-, Cedernholz- und Lavendelöl weisen eine insektenabweisende Wirkung auf.

Riechstoffe sind vielfach leichtflüchtig und unbeständig gegen Oxidationsmittel, sie verändern sich häufig bereits an Luft und im Licht. Riechstoffe lassen sich untergliedern in leicht flüchtige, flüchtige und gering flüchtige Riechstoffe. Beispiele für gering flüchtige Riechstoffe, also Stoffe mit hohem Siedepunkt, sind Benzophenon, Benzylsalicylat, Galaxolide, Derivate des Zimtaldehydes, Phenylethylphenylacetat. Beispiele für flüchtige Riechstoffe sind Zimtalkohol, Coumarin, Derivate der Salicylsäure, phenolische Riechstoffe (Vanillin, Eugenol), Cedryl-Derivate. Beispiele für hoch flüchtige Riechstoffe sind Aldehyde, Alkohole sowie Ester von Terpenen (Citral, Linalool, Geranylacetat). Als Riechstoffe geeignete etherische Öle sind beispielsweise Agrumenöle (Zitronen-, Orangenöl), Öle von Kräutern (Rosmarin-, Thymian-, Oreganumöl), Öle von Hölzern (Rosenholzöl, Cedernholzöl). Diese Öle können, basierend auf der Wirkung einzelner Komponenten, eine biozide, fungizide sowie durch ihren Geruch Insekten abweisende Wirkung aufweisen.

Wegen der Flüchtigkeit von Riechstoffen ist ihr an sich erwünschter breiter Einsatz eingeschränkt. Wünschenswert ist der Einsatz von Riechstoffen in zahlreichen Dispersionen, u. a. in Anstrichfarbe oder Mörtel basierend auf Bindemitteln bzw. entsprechenden Dispersionen.

In der Schrift US 6740692 B2 werden Cyclodextrine zur Stabilisierung von Fungiziden sowie Photoinitiatoren beschrieben.

In der Patentschrift EP 0186146 B1 werden Einschlussverbindungen von Wirkstoffen mit Cyclodextrinen in Verbindung mit einem reduzierten Cyclodextrin-Hirse-Brei beschrieben. Die eingesetzte CD-Wirkstoffmischung hat ein molares Verhältnis CD:Wirkstoff von 0,15 - 0,2:1.

Aufgabe der vorliegenden Erfindung ist es, eine riechstoffhaltige Dispersion zur Verfügung zu stellen, die Riechstoffe in einer gegenüber Flüchtigkeit, oxidativer und UV bedingter Zersetzung stabilisierten Form enthält und diese über einen langen Zeitraum, beispielsweise mehrere Jahre, verzögert freisetzt.

Diese Aufgabe wird gelöst durch eine lösungsmittelfrei herstellbare Dispersion enthaltend folgende Substanzen
a) mindestens ein filmbildendes Polymerisat von einem oder mehreren Monomeren aus der Gruppe umfassend Vinylester, (Meth)acrylsäureester, Vinylaromaten, Olefine, 1,3-Diene und Vinylhalogenide und gegebenenfalls weiteren, damit copolymerisierbaren Monomeren, in Form dessen wässriger Polymerdispersion oder in Wasser redispergierbaren Polymerpulvers,
b) einen 1:1- oder 2:1-Cyclodextrin-Riechstoff-Komplex, wobei das Cyclodextrin ausgewählt ist aus der Gruppe α-Cyclodextrin, β-Cyclodextrin, γ-Cyclodextrin, Methyl-β-Cyclodextrin, Methyl-γ-Cyclodextrin, Hydroxypropyl-β-Cyclodextrin und Hydroxypropyl-γ-Cyclodextrin
c) ein Pigment,
d) ein oder mehrere Füllstoffe sowie gegebenenfalls
e) weitere Zusatzstoffe,
wobei das Polymerisat a) in Form einer Polymerdispersion oder eines Polymerpulvers mit den weiteren Rezepturbestandteilen b) bis e) in geeigneten Mischern, ohne Zusatz von nichtwässrigen, flüchtigen Lösungsmitteln, gemischt und homogenisiert wird.

Eine erfindungsgemäße Dispersion umfasst somit auch Mischungen der genannten Komplexe. Unter lösungsmittelfrei herstellbar ist dabei zu verstehen, dass die Zugabe der Riechstoffe ohne Zusatz von Lösungsmittel erfolgen kann und damit lösungsmittelfreie bzw. emissionsarme Beschichtungsmittel zugänglich werden, deren Anteil an flüchtigen, nichtwässrigen Bestandteilen kleiner 1 Gew.-% beträgt, bezogen auf das Gesamtgewicht der Dispersion.

Vorzugsweise ist das Cyclodextrin ausgewählt aus der Gruppe α-Cyclodextrin, β-Cyclodextrin, γ-Cyclodextrin und Methyl-β-Cyclodextrin.

Überraschend zeigte sich, dass erfindungsgemäße Dispersionen die genannten Komponenten sowie Komplexe von α-, β- oder γ-Cyclodextrin sowie deren genannten Derivaten mit Riechstoffen im Molverhältnis 1:1 sowie 2:1 enthalten, eine deutlich erhöhte Geruchsstabilität, verbunden mit einer bioziden (fungi-, bakteri-, germi-ziden) Wirkung über die Feuchte kontrollierten Freisetzung von Riechstoffen im Vergleich zu Dispersionen mit nicht komplexierten Riechstoffen aufweisen. Der Gehalt an Riechstoff bleibt in diesen Dispersionen nach Lagerung unter Luft und Tageslicht konstant und ist damit beispielsweise in Anstrichprodukten im gewünschten Maß verfügbar. Dieser Effekt tritt bei den aus EP 0186146 B1 bekannten CD-Wirkstoffmischungen mit einem molaren Verhältnis CD : Wirkstoff von 0,15 - 0,2 : 1 nicht auf. Derartige CD-Wirkstoffmischungen weisen keine biozide (fungi-, bakteri-, germi-ziden) Wirkung auf.

Zur Herstellung eines in der erfindungsgemäßen Dispersion vorhanden Komplexes wird 1 mol Riechstoff von 1 bzw. 2 mol α-Cyclodextrin, β- bzw. γ-Cyclodextrin oder deren Methyl- sowie Hydroypropyl-Derivaten komplexiert.

Die im erfindungsgemäßen Komplex eingesetzten Riechstoffe sind synthetischen, halbsynthetischen oder natürlichen Ursprungs.

Sie lassen sich nach folgenden Produktgruppen unterscheiden:
- Synthetische Riechstoffe, vorzugsweise Terpenverbindungen, welche in der Pflanzenwelt weit verbreitet sind; Aromaten wie Phenylethylalkohol, Cyclamenaldehyd und Galaoxid, Benzaldehyd sowie Zimtaldehyd, a-Amyl- und α-Hexylzimtaldehyd, Menthol, Thymol, Moschus Ambrette, Cumarin, Salicylaldehyd, Anethol, Anisol, Salicylsäureester, Vanillin, Isoeugenol, Sandel; Aliphaten, wie Fettaldehyde, -ester, -alkohole, Alicyclen (Cycloaliphaten), wie Jasminriechstoffe beispielsweise cis-Jasmon, Dihydromethyljasmonat; makrocyclische Ketone (Muscon und Zibeton); Heterocyclen, wie Lactone (z. B. Cyclopentadecanolid); Stickstoff- oder Schwefelheterocyclen (Pyridine, Pyrazine, Thiophenew, Thiazole u. a.);
- Halbsynthetische Riechstoffe z. B. erhältlich aus Isolaten durch chemische Umsetzung, z. B. 1-Carvon aus Limonen, Cedrylacetat aus Cedrol, Hydroxycitronellal aus Citronellal oder Terpineol aus Pinien;
- Naturstoffe die mit Hilfe physikalischer oder chemischer Trennmethoden aus ätherischen Ölen gewonnen werden, z. B. Eugenol aus Nelkenblätteröl, Eucalyptol aus Eucalyptusöl globolus, Cedrol aus Cedernholzöl, Citral aus Lemongrasöl und 1-Menthol aus Pfefferminzöl.
- Ätherischen Öle, wie Citrus-, Rosmarin-, Teebaum-, Thymian-, Cedernholz-, Lavendel-, Pine-, Nelkenblüten-, Lemongrasöl Aie sind Naturstoffe und werden aus Gräsern, Gewürzen, Fruchtschalen durch u. a. Auspressen gewonnen. Diese Öle können neben dem Duft über eine bakterizide, fungizide Repellant-Wirkung verfügen. Neben Naturstoffen können auch synthetische Stoffe wie DEET eingesetzt werden.
- Parfums: Sie weisen die Klassifizierung nach Duftfamilien wie folgt auf:
   Grün-Noten beschreiben die Geruchsvorstellung von Blättern, Wiesen oder Gras und sind zudem unterteilt in frisch und balsamisch, in leichte, kühle Noten von Citrone und Kräutern sowie der grünen Richtung und in die weichen, warmen Noten aus Naturprodukten, blumigen Noten wie Maiglöckchen (Hydroxycitronelal, Lilial, Lyral), Rose (Phenylethylalkohol, Geraniol), Flieder (Terpineol) sind unterteilt in die Richtungen fruchtig-frisch, blumig-frisch, blumig und blumig-süß, Aldehyd-Noten (C7-C13-Aldehyde) bestehen aus rein synthetisch hergestellten Riechstoffen mit ausgeprägtem aldehydigblumigen-holzig-pudrig Geruch, Chypre-Noten (Eichenmoos, Evernyl), sind betont in die Richtungen frisch-moosigaldehydig sowie animalisch und fruchtig unterteilt, orientalischen Noten, mit ausgeprägter schwerer Süße erzielt durch verschiedene Harze und tierische Duftstoffe, Tabak-/Leder-Noten (Kresole), sind Phantasieakkorde mit teerigen und tierischen Elementen, Fougère-Noten mit Tabak-Leder und krautig-frischen, von Lavendel geprägten Noten.

Vorzugsweise handelt es sich bei den Riechstoffen um Riechstoffe ausgewählt aus der Gruppe von Riechstoffen mit einer bioziden (fungi-, bakteri-, germi-ziden) Wirkung oder Riechstoffe mit einer Wirkung als Repellent.

Die Riechstoffe-Komplexe mit genannten α-/β-/γ-Cyclodextrinen bzw. den genannten CD-Derivaten können in bekannter Weise aus z. B. Lösung oder mit der Pastenmethode hergestellt werden.

Die verwendeten Einsatzstoffe sind vorzugsweise:
Citral, Molekulargewicht 152,24 g/mol (3,7-Dimethyl-2,6-octadienal; Parfums mit einem durchschnittlichen Molekulargewicht von 180 g/mol, Bergamottöl durchschnittliches Molekulargewicht 170 g/mol, Teebaumöl durchschnittliches Molekulargewicht 145 g/mol, Cedernholzöl durchschnittliches Molekulargewicht 230 g/mol, DEET Molekulargewicht 191 g/mol, α-Cyclodextrin, Molekulargewicht 973 g/mol β-Cyclodextrin, Molekulargewicht 1135 g/mol erhältlich unter der Bezeichnung CAVAMAX® W7 bei der Firma Wacker Chemie AG, München, γ-Cyclodextrin, Molekulargewicht 1297 g/mol erhältlich unter der Bezeichnung CAVAMAX® W8 bei der Firma Wacker Chemie AG, München.

Als vorteilhaft hat sich die Herstellung der Komplexe aus einer konzentrierten, wässrigen Cyclodextrin-Zubereitung erwiesen. Die Cyclodextrin Konzentration der Zubereitung liegt zwischen 5 - 50 Gewichts-%. Bevorzugt ist eine Cyclodextrin-Konzentration von 20 - 50 %. Das Gewichts-Verhältnis (α-, β- bzw. γ-Cyclodextrin zum Riechstoff) liegt zwischen 9,0:1,0 und 5,0:1,0. Die Ansätze werden je nach Konsistenz intensiv gerührt oder geknetet.

Die Reaktionstemperatur liegt vorzugsweise bei 20 - 80°C. Bevorzugt wird bei 20 - 70°C, besonders bevorzugt bei 50 - 65°C gearbeitet. Die Reaktionsdauer hängt von der Temperatur ab und liegt zwischen einer Stunde und einigen Tagen. Bevorzugt ist eine Reaktionszeit von 3 bis 30 Stunden.
Die Komplexierung erfolgt in der Regel unter Normaldruck. Bevorzugt findet die Komplexierung unter Schutzgasatmosphäre (Stickstoff oder Argon) sowie Ausschluss von Tageslicht statt.

Die kaum wasserlöslichen Komplexe können direkt verwendet werden. Sie können aber auch durch Filtration, Zentrifugation, Trocknung, Mahlen, Sieben, Sichten, Granulieren, Tablettieren entsprechend isoliert und aufbereitet werden.

Geeignete Vinylester sind solche von Carbonsäuren mit 1 bis 12 C-Atomen. Bevorzugt werden Vinylacetat, Vinylpropionat, Vinylbutyrat, Vinyl-2-ethylhexanoat, Vinyllaurat, 1-Methylvinylacetat, Vinylpivalat und Vinylester von α-verzweigten Monocarbonsäuren mit 9 bis 11 C-Atomen, beispielsweise VeoVa9^{®} oder VeoVa10® (Handelsnamen der Firma Shell) eingesetzt. Besonders bevorzugt ist Vinylacetat.

Geeignete Monomeren aus der Gruppe Acrylsäureester oder Methacrylsäureester sind Ester von unverzweigten oder verzweigten Alkoholen mit 1 bis 15 C-Atomen. Bevorzugte Methacrylsäureester oder Acrylsäureester sind Methylacrylat, Methylmethacrylat, Ethylacrylat, Ethylmethacrylat, Propylacrylat, Propylmethacrylat, n-Butylacrylat, n-Butylmethacrylat, t-Butylacrylat, t-Butylmethacrylat, 2-Ethylhexylacrylat. Besonders bevorzugt sind Methylacrylat, Methylmethacrylat, n-Butylacrylat, t-Butylacrylat und 2-Ethylhexylacrylat.

Als Vinylaromaten bevorzugt sind Styrol, Methylstyrol und Vinyltoluol. Bevorzugtes Vinylhalogenid ist Vinylchlorid. Die bevorzugten Olefine sind Ethylen, Propylen und die bevorzugten Diene sind 1,3-Butadien und Isopren.

Gegebenenfalls können noch 0,1 bis 5 Gew.-%, bezogen auf das Gesamtgewicht des Monomergemisches, Hilfsmonomere copolymerisiert werden. Bevorzugt werden 0.5 bis 2.5 Gew.-% Hilfsmonomere eingesetzt. Beispiele für Hilfsmonomere sind ethylenisch ungesättigte Mono- und Dicarbonsäuren, vorzugsweise Acrylsäure, Methacrylsäure, Fumarsäure und Maleinsäure; ethylenisch ungesättigte Carbonsäureamide und -nitrile, vorzugsweise Acrylamid und Acrylnitril; Mono- und Diester der Fumarsäure und Maleinsäure wie die Diethyl- und Diisopropylester sowie Maleinsäureanhydrid, ethylenisch ungesättigte Sulfonsäuren bzw. deren Salze, vorzugsweise Vinylsulfonsäure, 2-Acrylamido-2-methyl-propansulfonsäure. Weitere Beispiele sind vorvernetzende Comonomere wie mehrfach ethylenisch ungesättigte Comonomere, beispielsweise Divinyladipat, Diallylmaleat, Allylmethacrylat oder Triallylcyanurat, oder nachvernetzende Comonomere, beispielsweise Acrylamidoglykolsäure (AGA), Methylacrylamidoglykolsäuremethylester (MAGME), N-Methylolacrylamid (NMA), N-Methylolmethacrylamid, N-Methylolallylcarbamat, Alkylether wie der Isobutoxyether oder Ester des N-Methylolacrylamids, des N-Methylolmethacrylamids und des N-Methylolallylcarbamats. Geeignet sind auch epoxidfunktionelle Comonomere wie Glycidylmethacrylat und Glycidylacrylat. Weitere Beispiele sind siliciumfunktionelle Comonomere, wie Acryloxypropyltri(alkoxy)- und Methacryloxypropyltri(alkoxy)-Silane, Vinyltrialkoxysilane und Vinylmethyldialkoxysilane,
wobei als Alkoxygruppen beispielsweise Ethoxy- und Ethoxypropylenglykolether-Reste enthalten sein können. Genannt seien auch Monomere mit Hydroxy- oder CO-Gruppen, beispielsweise Methacrylsäure- und Acrylsäurehydroxyalkylester wie Hydroxyethyl-, Hydroxypropyl- oder Hydroxybutylacrylat oder -methacrylat sowie Verbindungen wie Diacetonacrylamid und Acetylacetoxyethylacrylat oder -methacrylat.

Die Monomerauswahl bzw. die Auswahl der Gewichtsanteile der Comonomere erfolgt dabei so, dass im allgemeinen eine Glasübergangstemperatur Tg von -30°C bis +40°C, vorzugsweise -10°C bis +25°C resultiert. Die Glasübergangstemperatur Tg der Polymerisate kann in bekannter Weise mittels Differential Scanning Calorimetry (DSC) ermittelt werden. Die Tg kann auch mittels der Fox-Gleichung näherungsweise vorausberechnet werden. Nach Fox T. G., Bull. Am. Physics Soc. 1, 3, page 123 (1956) gilt: 1/Tg = x₁/Tg₁ + x₂/Tg₂ + ... + xₙ/Tgₙ, wobei xₙ für den Massebruch (Gew.-%/100) des Monomeren n steht, und Tgₙ die Glasübergangstemperatur in Kelvin des Homopolymeren des Monomeren n ist. Tg-Werte für Homopolymerisate sind in Polymer Handbook 2nd Edition, J. Wiley & Sons, New York (1975) aufgeführt.

Bevorzugt eingesetzt werden Homo- oder Mischpolymerisate, welche ein oder mehrere Monomere aus der Gruppe Vinylacetat, Vinylester von α-verzweigten Monocarbonsäuren mit 9 bis 11 C-Atomen, Vinylchlorid, Ethylen, Methylacrylat, Methylmethacrylat, Ethylacrylat, Ethylmethacrylat, Propylacrylat, Propylmethacrylat, n-Butylacrylat, n-Butylmethacrylat, 2-Ethylhexylacrylat, Styrol enthalten. Besonders bevorzugt eingesetzt werden Mischungen mit Vinylacetat und Ethylen; mit Vinylacetat, Ethylen und einem Vinylester von α-verzweigten Monocarbonsäuren mit 9 bis 11 C-Atomen; mit n-Butylacrylat und 2-Ethylhexylacrylat und/oder Methylmethacrylat; mit Styrol und einem oder mehreren Monomeren aus der Gruppe Methylacrylat, Ethylacrylat, Propylacrylat, n-Butylacrylat, 2-Ethylhexylacrylat; mit Vinylacetat und einem oder mehreren Monomeren aus der Gruppe Methylacrylat, Ethylacrylat, Propylacrylat, n-Butylacrylat, 2-Ethylhexylacrylat und gegebenenfalls Ethylen; mit 1,3-Butadien und Styrol und/oder Methylmethacrylat sowie gegebenenfalls weiteren Acrylsäureestern; wobei die genannten Gemische gegebenenfalls noch ein oder mehrere der obengenannten Hilfsmonomere enthalten können.

Am meisten bevorzugt eingesetzt werden Polymerisate der obengenannten Zusammensetzungen, welche noch 0,1 bis 5 Gew.-%, bezogen auf das Gesamtgewicht des Polymerisats, Monomereinheiten enthalten, welche sich ableiten von einem oder mehreren Comonomeren aus der Gruppe umfassend ethylenisch ungesättigte Mono- und Dicarbonsäuren wie Acrylsäure, Methacrylsäure, Fumarsäure und Maleinsäure; ethylenisch ungesättigte Carbonsäureamide und -nitrile wie Acrylamid und Acrylnitril; Monoester der Fumarsäure und Maleinsäure, sowie Maleinsäureanhydrid, ethylenisch ungesättigte Sulfonsäuren bzw. deren Salze, vorzugsweise Vinylsulfonsäure, 2-Acrylamido-2-methyl-propansulfonsäure.

Die Herstellung der Polymerisate a) erfolgt nach dem Emulsionspolymerisationsverfahren oder nach dem Suspensionspolymerisationsverfahren in Gegenwart von Schutzkolloiden und/oder Emulgatoren, vorzugsweise nach dem Emulsionspolymerisationsverfahren, wobei die Polymerisationstemperatur im allgemeinen 40°C bis 100°C, vorzugsweise 60°C bis 90°C beträgt, und bei der Copolymerisation von gasförmigen Comonomeren wie Ethylen auch unter Druck, im allgemeinen zwischen 5 bar und 100 bar, gearbeitet werden kann. Die Initiierung der Polymerisation erfolgt mit den für die Emulsionspolymerisation bzw. Suspensionspolymerisation gebräuchlichen wasserlöslichen bzw. monomerlöslichen Initiatoren oder Redox-Initiator-Kombinationen. Beispiele für wasserlösliche Initiatoren sind die Natriumpersulfat, Wasserstoffperoxid, Azobisisobutyronitril. Beispiele für monomerlösliche Initiatoren sind Dicetylperoxydicarbonat, Dicyclohexylperoxydicarbonat, Dibenzoylperoxid. Die genannten Initiatoren werden im allgemeinen in einer Menge von 0.01 bis 0.5 Gew.-%, bezogen auf das Gesamtgewicht der Monomere, eingesetzt. Als Redox-Initiatoren verwendet man Kombinationen aus den genannten Initiatoren in Kombination mit Reduktionsmitteln. Geeignete Reduktionsmittel sind beispielsweise Natriumsulfit, Natriumhydroxymethansulfinat und Ascorbinsäure. Die Reduktionsmittelmenge beträgt vorzugsweise 0.01 bis 0.5 Gew.-%, bezogen auf das Gesamtgewicht der Monomere.

Zur Steuerung des Molekulargewichts können während der Polymerisation regelnde Substanzen eingesetzt werden. Falls Regler eingesetzt werden, werden diese üblicherweise in Mengen zwischen 0,01 bis 5,0 Gew.-%, bezogen auf die zu polymerisierenden Monomeren, eingesetzt und separat oder auch vorgemischt mit Reaktionskomponenten dosiert. Beispiele solcher Substanzen sind n-Dodecylmercaptan, tert.-Dodecylmercaptan, Mercaptopropionsäure, Mercaptopropionsäuremethylester, Isopropanol und Acetaldehyd. Vorzugsweise werden keine regelnden Substanzen verwendet.

Geeignete Schutzkolloide sind teilverseifte oder vollverseifte Polyvinylalkohole; Polyvinylpyrrolidone; Polyvinylacetale; Polysaccharide in wasserlöslicher Form, wie Stärken (Amylose und Amylopectin), Cellulosen und deren Carboxymethyl-, Methyl-, Hydroxyethyl-, Hydroxypropyl-Derivate; Proteine wie Casein oder Caseinat, Sojaprotein, Gelatine; Ligninsulfonate; synthetische Polymere, wie Poly(meth)acrylsäure, Copolymerisate von (Meth)acrylaten mit carboxylfunktionellen Comonomereinheiten, Poly(meth)acrylamid, Polyvinylsulfonsäuren und deren wasserlöslichen Copolymere; Melaminformaldehydsulfonate, Naphthalinformaldehydsulfonate, Styrolmaleinsäure- und Vinylethermaleinsäure-Copolymere. Bevorzugt werden teilverseifte oder vollverseifte Polyvinylalkohole. Besonders bevorzugt sind teilverseifte Polyvinylalkohole mit einem Hydrolysegrad von 80 bis 95 Mol% und einer Höpplerviskosität in 4 %iger wässriger Lösung von 1 bis 30 mPas (Methode nach Höppler bei 20°C, DIN 53015).

Geeignete Emulgatoren, die in 0,5 bis 10 Gew.-% bezogen auf die Monomermenge, eingesetzt werden können sind sowohl anionische, kationische als auch nichtionische Emulgatoren, beispielsweise anionische Tenside, wie Alkylsulfate mit einer Kettenlänge von 8 bis 18 C-Atomen, Alkyl- oder Alkylarylethersulfate mit 8 bis 18 C-Atomen im hydrophoben Rest und bis zu 40 Ethylen- oder Propylenoxideinheiten, Alkyl- oder Alkylarylsulfonate mit 8 bis 18 C-Atomen, Ester und Halbester der Sulfobernsteinsäure mit einwertigen Alkoholen oder Alkylphenolen, oder nichtionische Tenside wie Alkylpolyglykolether oder Alkylarylpolyglykolether mit 8 bis 40 Ethylenoxid-Einheiten.

Nach Abschluss der Polymerisation kann zur Restmonomerentfernung in Anwendung bekannter Methoden nachpolymerisiert werden, beispielsweise durch mit Redoxkatalysator initiierter Nachpolymerisation. Flüchtige Restmonomere können auch mittels Destillation, vorzugsweise unter reduziertem Druck und gegebenenfalls unter Durchleiten oder Überleiten von inerten Schleppgasen wie Luft, Stickstoff oder Wasserdampf entfernt werden.

Die mit dem erfindungsgemäßen Verfahren erhältlichen wässrigen Dispersionen haben einen Feststoffgehalt von 30 bis 75 Gew.-%, vorzugsweise von 50 bis 60 Gew.-%. Zur Herstellung der in Wasser redispergierbaren Polymerpulver werden die wässrigen Dispersionen, gegebenenfalls nach Zusatz von Schutzkolloiden, als Verdüsungshilfe, getrocknet, beispielsweise mittels Wirbelschichttrocknung, Gefriertrocknung oder Sprühtrocknung. Vorzugsweise werden die Dispersionen sprühgetrocknet. Die Sprühtrocknung erfolgt dabei in üblichen Sprühtrocknungsanlagen,
wobei die Zerstäubung mittels Ein-, Zwei- oder Mehrstoffdüsen oder mit einer rotierenden Scheibe erfolgen kann. Die Austrittstemperatur wird im allgemeinen im Bereich von 45°C bis 120°C, bevorzugt 60°C bis 90°C, je nach Anlage, Tg des Harzes und gewünschtem Trocknungsgrad, gewählt.

In der Regel wird die Verdüsungshilfe in einer Gesamtmenge von 3 bis 30 Gew.-%, bezogen auf die polymeren Bestandteile der Dispersion, eingesetzt. Das heißt, die Gesamtmenge an Schutzkolloid vor dem Trocknungsvorgang soll mindestens 3 bis 30 Gew.-%, bezogen auf den Polymeranteil betragen; bevorzugt werden 5 bis 20 Gew.-% bezogen auf den Polymeranteil eingesetzt.

Geeignete Verdüsungshilfen sind beispielsweise die bereits genannten Schutzkolloide. Bevorzugt werden keine weiteren Schutzkolloide als Polyvinylalkohole als Verdüsungshilfe eingesetzt. Bei der Verdüsung hat sich vielfach ein Gehalt von bis zu 1,5 Gew.-% Antischaummittel, bezogen auf das Basispolymerisat, als günstig erwiesen. Zur Erhöhung der Lagerfähigkeit durch Verbesserung der Verblockungsstabilität, insbesondere bei Pulvern mit niedriger Glasübergangstemperatur, kann das erhaltene Pulver mit einem Antiblockmittel (Antibackmittel), vorzugsweise bis 30 Gew.-%, bezogen auf das Gesamtgewicht polymerer Bestandteile, ausgerüstet werden. Beispiele für Antiblockmittel sind Ca- bzw. Mg-Carbonat, Talk, Gips, Kieselsäure, Kaoline, Silicate mit Teilchengrößen vorzugsweise im Bereich von 10 nm bis 10 µm.

Die Viskosität der zu verdüsenden Speise wird über den Feststoffgehalt so eingestellt, dass ein Wert von < 500 mPas (Brookfield-Viskosität bei 20 Umdrehungen und 23°C), bevorzugt < 250 mPas, erhalten wird. Der Feststoffgehalt der zu verdüsenden Dispersion beträgt > 35 %, bevorzugt > 40 %.

Vorzugsweise werden die CD-Riechstoffkomplexe b) in einer Menge von 1 bis 50 Gew.-%, vorzugsweise 5 bis 15 Gew.-%, jeweils bezogen auf den Polymerisatanteil a) eingesetzt.

Geeignete Pigmente c) sind dem Fachmann bekannt. Es können sowohl anorganische Pigmente als auch organische Pigmente eingesetzt werden. Beispiele für anorganische Weißpigmente sind Titandioxid, Zinkoxid, Zinksulfid, Bleicarbonat und Bariumsulfat. Beispiele für anorganische Farbpigmente sind Eisenoxidgelb, -rot und -schwarz, Ruß, Graphit, Chrom-, Cadmiumgelb bzw. -orange, Molybdatorange und -rot, Kobalt-, Eisen- und Ultramarinblau, Chromoxidgrün und Mischphasengrünpigmente mit Spinellstruktur und Manganviolett. Geeignete organische Farbpigmente sind Azo-, Antrachinon-, Chinacridon-, Phthalocyanin-, Perylen- und Indigo-Farbstoffe. Der Pigmentanteil ist abhängig von der Deckkraft des Pigments und der Farbtiefe und beträgt im allgemeinen 1 bis 30 Gew.-%, bezogen auf das Gesamtgewicht der Beschichtungsmittel-Zusammensetzung.

Beispiele für einsetzbare Füllstoffe d) sind Carbonate wie Calciumcarbonat in Form von Dolomit, Calcit und Kreide. Weitere Beispiele sind Silikate, wie Magnesiumsilikat in Form von Talkum, oder Aluminiumsilikate wie Kaolin, Glimmer, Lehm und Tone; Quarzmehl, Quarzsand, hochdisperse Kieselsäure, Feldspat, Schwerspat und Leichtspat. Geeignet sind auch Faserfüllstoffe natürlichen (Cellulosefaser) oder synthetischen (PE, Dralon) Ursprungs. In der Praxis werden häufig Gemische verschiedener Füllstoffe eingesetzt. Beispielsweise Gemische von Füllstoffen unterschiedlicher Teilchengröße oder Gemische von carbonatischen und silikatischen Füllstoffen. Kunststoffputze enthalten im allgemeinen grobkörnigere Füllstoffe als Dispersionsfarben. Die Körnung liegt dabei oftmals zwischen 0.2 und 5.0 mm. Der Füllstoffanteil beträgt im allgemeinen 5 bis 80 Gew.-%, bezogen auf das Gesamtgewicht der Beschichtungsmittel-Zusammensetzung.

Im allgemeinen wird der Pigment- und Füllstoffanteil so bemessen, dass eine Pigmentvolumenkonzentration PVK von > 10 % resultiert. Bei Außenfarben beträgt die PVK vorzugsweise 25 bis 70 %, und errechnet sich mit PVK (%) = (VP+F x 100) / (VP+F + VB) mit VP+F = Volumen Pigment + Füllstoff und VB = Volumen Bindemittel.

Zu den Zusatzstoffen e) zählen anorganische Bindemittel, wie Zement, Kalk und Gips. Weitere Zusatzstoffe e) sind Verdickungsmittel, beispielsweise Polysaccharide, wie Cellulose-ether und modifizierte Celluloseether, Stärkeether, Guar Gum oder Xanthan Gum, Schichtsilikate, Polycarbonsäuren, wie Polyacrylsäure und deren Teilester, Polyvinylalkohole, welche gegebenenfalls acetalisiert und/oder hydrophob modifiziert sein können, Casein und assoziativ wirkende Verdicker oder Bentonit als Beispiel für ein anorganisches Verdickungsmittel. Es können auch Gemische der genannten Verdickungsmittel eingesetzt werden. Bevorzugt werden die Celluloseether, die modifizierten Celluloseether, die Polyvinylalkohole welche gegebenenfalls acetalisiert und/oder hydrophob modifiziert sein können, sowie deren Gemische. Vorzugsweise werden 0,05 bis 2,5 Gew.-%, besonders bevorzugt 0,05 bis 0,5 Gew.-% Verdickungsmittel eingesetzt.

Beispiele für weitere Zusatzstoffe e) sind Netzmittel in Anteilen von im allgemeinen 0,1 bis 0,5 Gew.-%, bezogen auf das Gesamtgewicht der Rezeptur. Beispiele hierfür sind Natrium- und Kaliumpolyphosphate, Polyacrylsäuren und deren Salze. Weitere Zusatzstoffe sind Entschäumer und Gefrierschutzmittel.

Bei der erfindungsgemäßen Dispersion liegen die Riechstoffe in mit Cyclodextrin komplexierter Form vor.

Zur Herstellung der erfindungsgemäßen Dispersion wird das Polymerisat a) in Form einer Polymerdispersion oder eines Polymerpulver mit den weiteren Rezepturbestandteilen b) bis e) in geeigneten Mischern, ohne Zusatz von nichtwässrigen, flüchtigen Lösungsmitteln, gemischt und homogenisiert. Das Polymerpulver kann gegebenenfalls auch in Form einer wässrigen Redispersion auf der Baustelle zugegeben werden. In vielen Fällen wird eine Trockenmischung hergestellt und das zur Verarbeitung erforderliche Wasser unmittelbar vor der Verarbeitung hinzugefügt. Bei der Herstellung von pastösen Massen wird häufig zunächst der Wasseranteil vorgelegt, die Dispersion zugegeben und abschließend werden die Feststoffe eingerührt.

Bei der erfindungsgemäßen Dispersion handelt es sich vorzugsweise um ein Beschichtungsmittel, besonderes bevorzugt um eine Wandfarbe. Die Beschichtungsmittel-Zusammensetzung, die Komplexe von β- sowie γ-Cyclodextrin mit Riechstoffen im Molverhältnis 1:1 sowie 2:1 enthält, eignet sich somit zur Verwendung als Dispersionsfarbe, Dichtungsmasse oder Kunststoffputz. Besonders bevorzugt ist die Verwendung im Außenbereich, insbesondere auf mineralischen Untergründen. Die erfindungsgemäße Dispersion eignet sich ferner als Klebstoff, z. B. als Fußboden- oder Fliesenkleber, als Produkt bzw. Dispersion der Bauindustrie, wie Dichtungs- und Haftschlamm, Wärmedämmsystem, Hydrophobierung- und Konservierungsmittel, Putz, Fugenmörtel, Spachtelmasse, Farbe, Lack.

Die nachfolgenden Beispiele dienen der weiteren Erläuterung der Erfindung:
Die in den Beispielen verwendete SPME-Methode ermöglicht die Konzentrierung von flüchtigen und nichtflüchtigen Verbindungen aus wässrigen und gasförmigen Matrizes für die GC, GC/MS und HPLC-Analytik. Zur Anreicherung dient dabei eine ca. 1 cm lange fused silica Faser, welche mit verschiedenen polymeren Phasen, wie z. B. Polydimethylsiloxan (PDMS) oder Polyacrylat, beschichtet ist. Anschließend werden die angelagerten Verbindungen durch thermische Desorption im GC-injection port bzw. durch die mobile Phase bei der HPLC desorbiert. Die Vorteile der SPME sind neben der Einfachheit der Methodik vor allem Zeiteffizienz und das Fehlen von Lösemitteln zur Extraktion (GC). Das Verfahren ist im Stand der Technik bekannt. Bestimmung der "freigesetzten Riechstoffe" der Beschichtung:
   Für die SPME-Analyse werden 500 mg Wandfarbe von der Wand abgetragen, in ein Fläschchen eingewogen und verschlossen. Die Probe wird trocken sowie unter Zusatz von 100 mg H₂O vermessen.

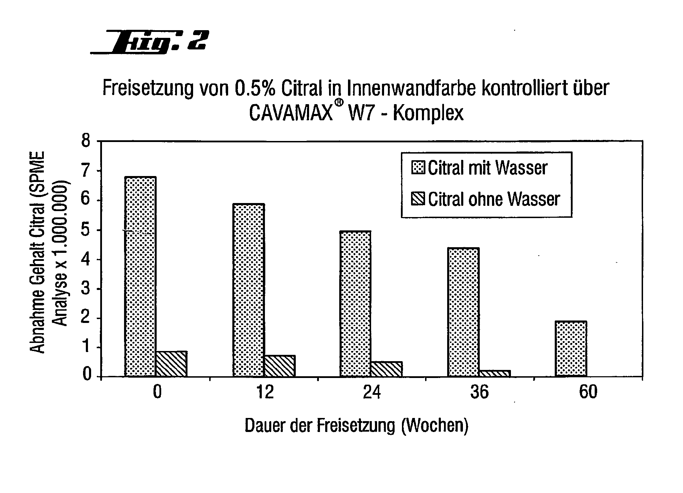

Die Ergebnisse sind in den Figuren 2, 4 und 7 aufgeführt.

Beispiel eines lösemittelfrei herstellbaren Beschichtungsmittels (Innenwandfarbe), welches in den Beispielen verwendet wurde:

**Tabelle 1:**

| Komponente | Funktion | Menge [g] |
|---|---|---|
| | | |
| Wasser | | 291.0 |
| Na-Polyacrylat (40 %-ig) | Dispergiermittel | 2.0 |
| Biozid | Topfkonservierung | 2,0 |
| Cellulose-Ether | Rheologisches Additiv | 2.0 |
| Acrylat-Verdicker | Rheologisches Additiv | 1.0 |
| Lusolvan FBH | Koaleszenzmittel | 3.0 |
| Cellulosefaser | Füllstoff | 5.0 |
| Faserfüllstoff | Füllstoff | 1.0 |
| Titandioxid | Pigment | 100.0 |
| Dispersion 50 %-ig | Bindemittel | 120.0 |
| Talkum(30 µm) | Füllstoff | 70.0 |
| Calciumcarbonat (50 µm) | Füllstoff | 250.0 |
| Calciumcarbonat (100 µm) | Füllstoff | 150.0 |
| Entschäumer | | 2.0 |
| Natronlauge (10%ig) | pH-Wert einstellen | 1.0 |
| | | |
| Summe | | 1000 |

### Beispiel 1: Komplexierung von Citral mit β-CD für einen Vergleich der kontrollierten Freisetzung

### 1:1-beta-Cyclodextrin-Komplex

1500 g β-Cyclodextrin trocken (Wassergehalt 10 %) wurden mit 1500 ml H₂O unter Rühren vermischt und auf 70°C erwärmt. Während des Erwärmens auf 70°C wird 202 g Citral zugegeben. Die Suspension wird bei 70°C einen Tag gerührt und im Vakuum bei 35°C getrocknet.
Ausbeute: 1700 g (99.8 %), Feuchte: 4.5 %, Gehalt an Citral lt. NMR: 10 %

### Beispiel 2: Komplexierung von Bergamottöl mit β-CD für einen Vergleich der kontrollierten Freisetzung

### 1:1-beta-Cyclodextrin-Komplex

1400 g β-Cyclodextrin (Wassergehalt 12 %) wurden mit 1300 g H₂O unter Rühren auf 50°C erwärmt und 154.4 g Bergamottöl zugegeben. Der Ansatz wird 10h gerührt und bei 45°C unter Vakuum getrocknet.
Ausbeute: 1450 g (93.3 %), Feuchte: 6.5 %, Gehalt Bergamottöl lt. Headspace-Gaschromatographie: 10 %

### Beispiel 3: Komplexierung von Parfume SO3076 mit β-Cyclodextrin für einen Vergleich der kontrollierten Freisetzung

### 1:1-beta-Cyclodextrin-Komplex

130 g β-Cyclodextrin (Wassergehalt: 12 %) wurden mit 100 g H₂O unter Rühren auf 70°C erwärmt und dann 16 g Parfume SO3076 zugegeben. Der Ansatz wird 6h bei 70°C sowie 10 h bei Raumtemperatur gelagert und unter Normalbedingung getrocknet.
Ausbeute: 137 g (94 %), Feuchte:10 %, Gehalt an Parfume SO3076 lt. Headspace-Gaschromatographie: 11.7 %

### Rezeptur Parfume SO 3076:

Linalool 18 %, Benzil Acetat 9.8 %, Galoxolide 11.6 %, Phenyl Ethyl Alkohol 11.6 %, Hexyl Cinnamic Aldehyde 19.6 %, Lilial 29.4 %

### Beispiel 4: Komplexierung von Teebaumöl mit β-Cyclodextrin für einen Vergleich der kontrollierten Freisetzung

### 1:1-beta-Cyclodextrin-Komplex

2657 g β-Cyclodextrin (Wassergehalt: 10 %) wurden mit 4.700 ml H2O unter rühren auf 70°C erwärmt und 325 g Teebaumöl zugegeben. Der Ansatz wird 10 h bei 70°C gerührt, filtriert und bei 50°C unter Vakuum getrocknet.
Ausbeute: 2825 g (94.7 %), Feuchte: 6.96 %, Gehalt Teebaumöl lt. Headspace-Gaschromatographie: 10.6 %

### Beispiel 5: Komplexierung von Zedernholzöl mit γ-Cyclodextrin für einen Vergleich der kontrollierten Freisetzung

### 1:1-gamma-Cyclodextrin-Komplex

0,1 Mol bzw. 142.52 g γ-Cyclodextrin (Wassergehalt: 9 %) wurden mit 255 g H₂O unter Rühren auf 65°C erwärmt und mit 0,1 Mol bzw. 23 g Zedernholzöl 24 h weiter gerührt und bei 40°C unter Vakuum getrocknet.
Ausbeute: 165 g (99 %), Feuchte: 10 %, Gehalt an Zedernholzöl lt. Headspace-Gaschromatographie 14 %

### Beispiel 6: Komplexierung von DEET mit β-Cyclodextrin für einen Vergleich der kontrollierten Freisetzung

### 1:1-beta-Cyclodextrin-Komplex

386.93 g β-Cyclodextrin (Wassergehalt: 10 %) wurden mit 900 ml H₂O und 57.38 g DEET bei 60°C 48h gerührt, filtriert und bei 50°C unter Vakuum getrocknet.
Ausbeute: 393.2 g (88 %), Feuchte: 6.93 %, Gehalt an DEET lt. NMR: 14.3 %

### Beispiel 7: Komplexierung von Phenylethylanthranilate mit β-Cyclodextrin für einen Vergleich der kontrollierten Freisetzung

### 2:1-beta-Cyclodextrin-Komplex

0,1 Mol bzw. 113,5 g β-Cyclodextrin (Wassergehalt: 14 %) wurden mit 68 ml H₂O und 0,05 Mol bzw. 12,065 g Phenylethylanthranilate untergerührt und 48 h bei 60°C gelagert, bei 50°C unter Vakuum getrocknet.
Ausbeute: 111,75 g (89 %), Feuchte: 10 %, Gehalt an Phenylethylanthranilate lt. NMR: 9.6 %

### Beispiel 8: Herstellung einer Beschichtung mit Riechstoffen bzw. mit 1:1- bzw. 2:1-β-,gamma-Cyclodextrin-Komplex

8a) Herstellung des Innwandbeschichtungsmittels mit einem Riechstoff (Citral, Fragrance SO 3076, Bergamottöl, Teebaumöl) (Vergleichsbeispiel):
   Dem Innwandbeschichtungsmittel (Dispersionfarbe) werden 0.5 % Riechstoff zugefügt und homogenisiert.
8b) Herstellung des Innwandbeschichtungsmittels mit einem Citral 1:1-beta-Cyclodextrin-Komplex:
   313,5 g Innwandbeschichtungsmittel (Dispersionfarbe) werden 16,5 g Komplex aus Bsp. 1 zugefügt und homogenisiert, damit ist der Gehalt an Citral in dem Innwandbeschichtungsmittel 0,5 %.
8c) Herstellung des Innwandbeschichtungsmittels mit einem Fragrance SO 3076 1:1-beta-Cyclodextrin-Komplex:
   315,9 g Innwandbeschichtungsmittel (Dispersionfarbe) werden 14,1 g Komplex aus Bsp. 3 zugefügt und homogenisiert, damit ist der Gehalt an Fragrance SO 3076 in dem Innwandbeschichtungsmittel 0,5 %.
8d) Herstellung des Innwandbeschichtungsmittels mit einem Bergamottöl 1:1-beta-Cyclodextrin-Komplex:
   313,5 g Innwandbeschichtungsmittel (Dispersionfarbe) werden 16,5 g Komplex aus Bsp. 2 zugefügt und homogenisiert, damit ist der Gehalt an Bergamottöl in dem Innwandbeschichtungsmittel 0,5 %.
8e) Herstellung des Innwandbeschichtungsmittels mit einem Teebaumöl 1:1-beta-Cyclodextrin-Komplex:
   314,4 g Innwandbeschichtungsmittel (Dispersionfarbe) werden 15,6 g Komplex aus Bsp. 4 zugefügt und homogenisiert, damit ist der Gehalt an Teebaumöl in dem Innwandbeschichtungsmittel 0,5 %.
8f) Herstellung des Innwandbeschichtungsmittels mit einem Phenylethylanthranilat 2:1-beta-Cyclodextrin-Komplex:
   94.8 g Innwandbeschichtungsmittel (Dispersionfarbe) werden 5.2 g Komplex aus Bsp. 7 zugefügt und homogenisiert, damit ist der Gehalt Phenylethylanthranilat an in dem Innwandbeschichtungsmittel 0,5 %.

Die Herstellung der Beschichtung mit den genannten Cyclodextrin-Komplexen erfolgte folgendermaßen:
Zu der Beschichtung aus Tab. 1 wird der Cyclodextrin-Komplex (10 % Riechstoff-Gehalt) gegeben und mit einem Rührer so lange gerührt, bis die Mischung homogen ist.
Auftragen der Beschichtung:
Auf einen m² Wand werden 150 g der jeweiligen Beschichtung gleichmäßig aufgetragen.

Der Komplex kann auch ein Bestandteil der in Tabelle 1 aufgezählten Komponenten mit den Funktionen wie folgt sein: Dispergiermittel (Na-Polyacrylat), Rheologisches Additiv (Cellulose-Ether, Acrylat-Verdicker), Füllstoff (Cellulosefaser, Faserfüllstoff, Talkum, Calciumcarbonat), Bindemittel (Polyvinylderivat), Entschäumer (Silicon). Er kann somit auch mittels der Komponenten mit unterschiedlicher Funktionalität der Dispersion zugesetzt werden.

### Beispiel 9: Bestimmung der Freisetzung von Citral als 1:1-beta-Cyclodextrin-Komplex in der Beschichtung

Beispiel 9a: Sensorische Bestimmung der Freisetzung von Citral als 1:1-beta-Cyclodextrin-Komplex in der Beschichtung

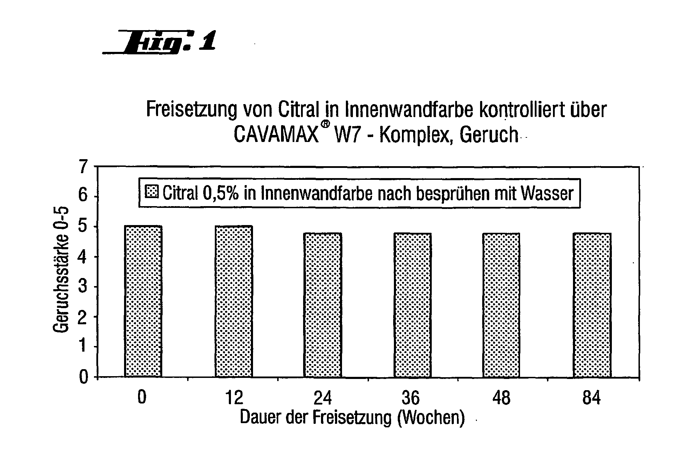

Ein m² Wandbeschichtung wurde monatlich mit 7.5 g Wasser besprüht und monatlich abgerochen. Die Geruchsskala 0 - 5 weist bei 0 kein Dufterlebnis bei 5 ein sehr starkes, dem Originalduft vergleichbares Dufterlebnis auf. Fig. 1 zeigt die Geruchsstärke des Zitronenduftes von Citral in den gelagerten Proben.

**Beispiel** 9b: Analytische Bestimmung der Freisetzung von Citral als 1:1-beta-Cyclodextrin-Komplex in der Beschichtung über SPME-Methode.

500 mg Wandbeschichtung wurde vom Exponat einer 1 m² Wandbeschichtung abgetragen und alle drei Monate über die SPME-Methode vermessen. Fig. 2 zeigt die flüchten Bestandteile im Gasraum der Probe vor und nach Zusatz von 100 mg Wasser.

### Beispiel 10: Bestimmung der Freisetzung von Fragrance SO 3076, Bergamottöl, Teebaumöl als 1:1-beta-Cyclodextrin-Komplex in der Beschichtung

Beispiel 10a: Sensorische Bestimmung der Freisetzung von Fragrance SO 3076, Bergamottöl, Teebaumöl als 1:1 beta-Cyclodextrin-Komplex in der Beschichtung.

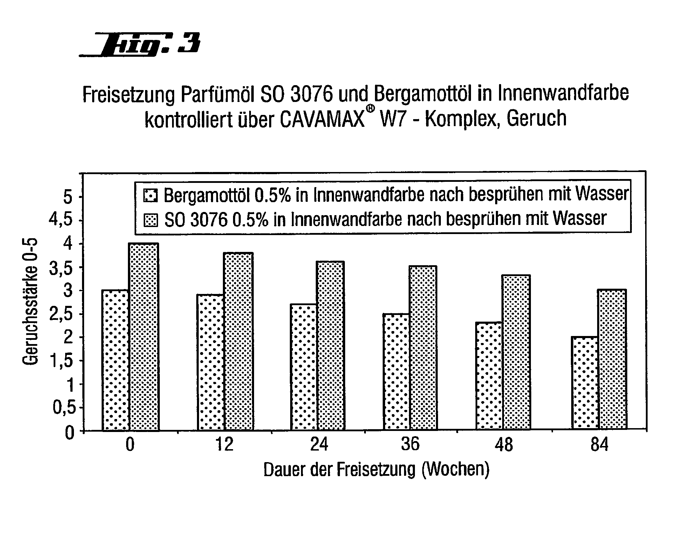

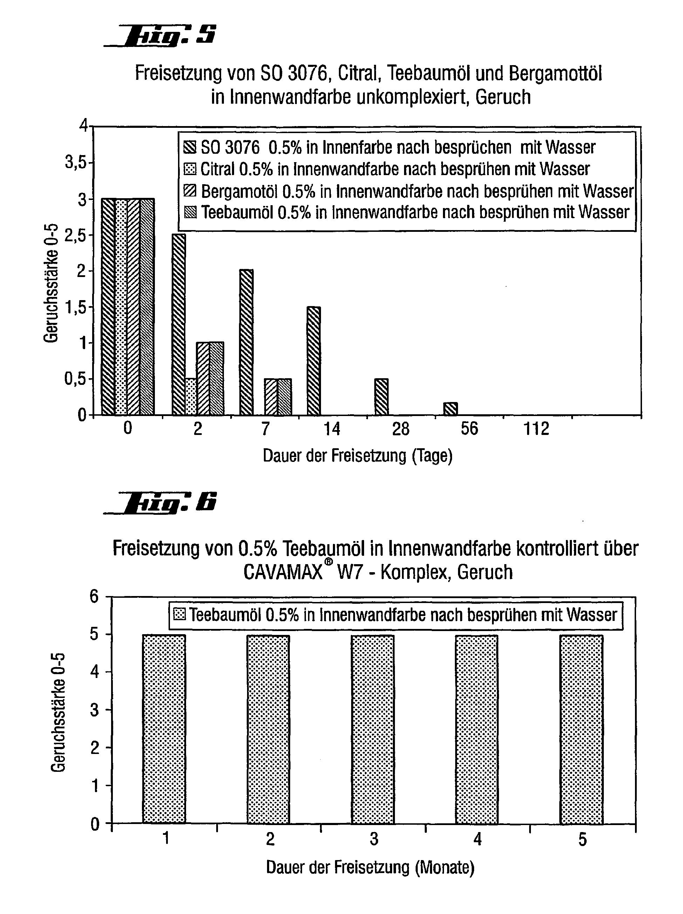

Eine ein m² Wandbeschichtung wurde monatlich mit 7,5 g Wasser besprüht und monatlich abgerochen.
Die Geruchsskale 0 - 5 weist bei 0 kein Dufterlebnis bei 5 ein sehr starkes, beim Originalduft vergleichbares Dufterlebnis auf. Fig. 3 und 6 zeigen die Geruchsstärke der drei Duftmuster in den gelagerten Proben.

Beispiel 10b: Analytischische Bestimmung der Freisetzung von Fragrance SO 3076, Bergamottöl, Teebaumöl als 1:1-beta-Cyclodextrin-Komplex in der Beschichtung über SPME-Methode.

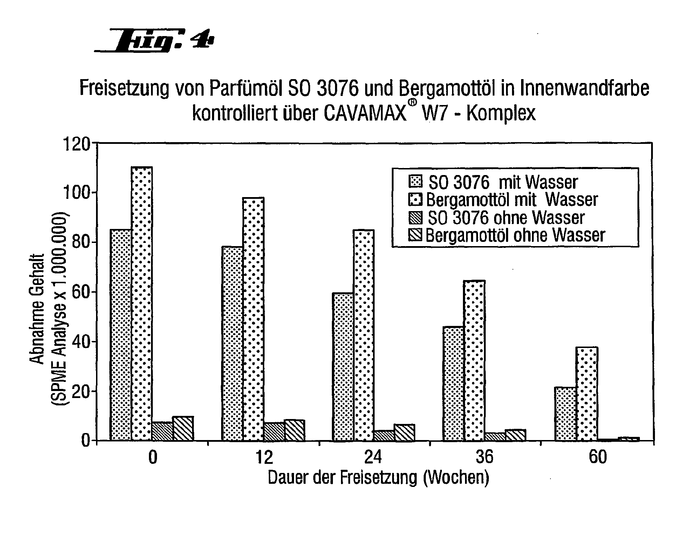

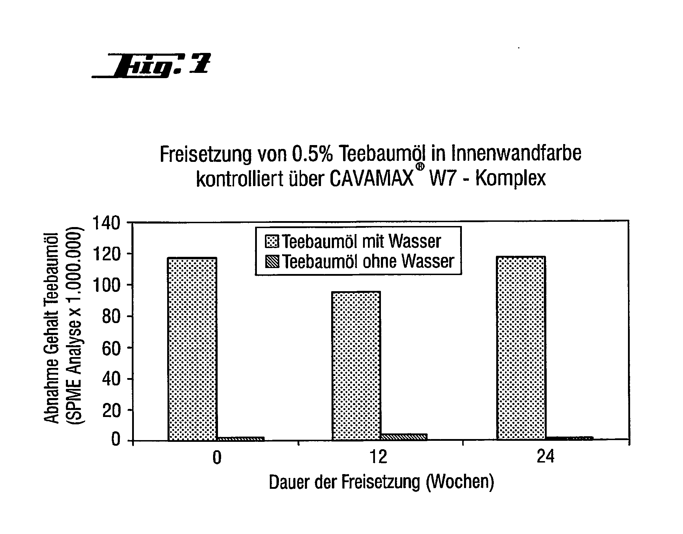

500 mg Wandbeschichtung wurde vom Exponat einer 1 m² Wandbeschichtung abgetragen und alle drei Monate über die SPME-Methode vermessen. Fig. 4 und 7 zeigen die flüchtigen Bestandteile im Gasraum der Probe vor und nach Zusatz von 100 mg Wasser.

### Beispiel 11: Sensorische Bestimmung der Freisetzung von Citral, Fragrance SO 3076, Bergamottöl, Teebaumöl unkomplexiert in der Beschichtung (Vergleichsbeispiel)

Eine ein m² Wandbeschichtung gemäß Beispiel 7a wurde bei Versuchsanfang täglich, dann wöchentlich mit 7.5 g Wasser besprüht und täglich, dann wöchentlich abgerochen. Die Geruchsskala 0 - 5 weist bei 0 kein Dufterlebnis, bei 5 ein sehr starkes, beim Originalduft vergleichbares Dufterlebnis auf.
Fig. 5 zeigt die Geruchsstärke des Duftes von Citral, Fragrance SO 3076, Bergamottöl, Teebaumöl in den gelagerten Proben.

## Patentansprüche

1. Lösungsmittelfrei herstellbare Dispersion enthaltend folgende Substanzen
a) mindestens ein filmbildendes Polymerisat von einem oder mehreren Monomeren aus der Gruppe umfassend Vinylester, (Meth)acrylsäureester, Vinylaromaten, Olefine, 1,3-Diene und Vinylhalogenide und gegebenenfalls weiteren, damit copolymerisierbaren Monomeren, in Form dessen wässriger Polymerdispersion oder in Wasser redispergierbaren Polymerpulvers,
b) einen 1:1- oder 2:1-Cyclodextrin-Riechstoff-Komplex, wobei das Cyclodextrin ausgewählt ist aus der Gruppe α-Cyclodextrin, β-Cyclodextrin, γ-Cyclodextrin, Methyl-β-Cyclodextrin, Methyl-γ-Cyclodextrin, Hydroxypropyl-β-Cyclodextrin und Hydroxypropyl-γ-Cyclodextrin
c) ein Pigment,
d) ein oder mehrere Füllstoffe sowie gegebenenfalls
e) weitere Zusatzstoffe,
wobei das Polymerisat a) in Form einer Polymerdispersion oder eines Polymerpulvers mit den weiteren Rezepturbestandteilen b) bis e) in geeigneten Mischern, ohne Zusatz von nichtwässrigen, flüchtigen Lösungsmitteln, gemischt und homogenisiert wird.

2. Dispersion gemäß Anspruch 1 **dadurch gekennzeichnet, dass** der Riechstoff ausgewählt ist aus der Gruppe von Riechstoffen mit einer bioziden (fungi-, bakteri-, germi-ziden) Wirkung.

3. Dispersion gemäß Anspruch 1 **dadurch gekennzeichnet, dass** der Riechstoff ausgewählt ist aus der Gruppe von Riechstoffen mit einer Wirkung als Repellent.

4. Dispersion gemäß Anspruch 3, welche als filmbildendes Polymerisat a) ein oder mehrere Homo- oder Mischpolymerisate enthält, welche ein oder mehrere Monomere aus der Gruppe Vinylacetat, Vinylester von α-verzweigten Monocarbonsäuren mit 9 bis 11 C-Atomen, Vinylchlorid, Ethylen, Methylacrylat, Methylmethacrylat, Ethylacrylat, Ethylmethacrylat, Propylacrylat, Propylmethacrylat, n-Butylacrylat, n-Butylmethacrylat, 2-Ethylhexylacrylat, Styrol enthält.

5. Dispersion gemäß Anspruch 3 oder 4, welche als filmbildendes Polymerisat a) ein oder mehrere Homo- oder Mischpolymerisate enthält, die noch 0,1 bis 5 Gew.-%, bezogen auf das Gesamtgewicht des Polymerisats, Monomereinheiten enthalten, welche sich ableiten von einem oder mehreren Comonomeren aus der Gruppe umfassend ethylenisch ungesättigte Mono- und Dicarbonsäuren, ethylenisch ungesättigte Carbonsäureamide und -nitrile, Monoester der Fumarsäure und Maleinsäure, sowie Maleinsäureanhydrid, ethylenisch ungesättigte Sulfonsäuren und deren Salze.

6. Dispersion nach Anspruch 3, 4 oder 5, welche CD-Riechstoff-komplex b) in einer Menge von 1 bis 50 Gew.-%, vorzugsweise 5 bis 15 Gew.-%, jeweils bezogen auf den Polymerisatanteil a) enthält.

7. Dispersion nach einem der Ansprüche 3 bis 6, bei dem Pigment- und Füllstoffanteil so bemessen ist, dass eine Pigmentvolumenkonzentration PVK von ≥ 10 % resultiert.

8. Verfahren zur Herstellung der Dispersion nach Anspruch 3 bis 7, **dadurch gekennzeichnet, dass** das Polymerisat a) in Form einer Polymerdispersion oder eines Polymerpulvers mit den weiteren Rezepturbestandteilen b) bis e) in geeigneten Mischern, ohne Zusatz von nichtwässrigen, flüchtigen Lösungsmitteln, gemischt und homogenisiert wird.

9. Verwendung einer Dispersion nach Anspruch 1 bis 7 als Beschichtungsmittel, in Dispersionsfarben, Dichtungsmassen oder Kunststoffputzen.

10. Verwendung einer Dispersion nach Anspruch 1 bis 7 in Dispersionsfarben, Dichtungsmassen oder Kunststoffputzen im Außenbereich.

## Claims

1. Solventlessly preparable dispersion comprising substances as follows:
a) at least one film-forming polymer of one or more monomers from the group encompassing vinyl esters, (meth)acrylic esters, vinylaromatics, olefins, 1,3-dienes and vinyl halides and optionally further monomers copolymerizable therewith, in the form of its aqueous polymer dispersion or water-redispersible polymer powder,
b) a 1:1 or 2:1 cyclodextrin-odorant complex, the cyclodextrin being selected from the group consisting of α-cyclodextrin, β-cyclodextrin, γ-cyclodextrin, methyl-β-cyclodextrin, methyl-γ-cyclodextrin, hydroxypropyl-β-cyclodextrin and hydroxypropyl-γ-cyclodextrin,
c) a pigment,
d) one or more fillers, and optionally
e) other additives,
the polymer a), in the form of a polymer dispersion or polymer powder, being mixed and homogenized with the other constituents, b) to e), of the formula, in suitable mixers, without addition of non-aqueous volatile solvents.

2. Dispersion according to Claim 1, **characterized in that** the odorant is selected from the group of odorants having a biocidal (fungi-, bacteri-, germi-cidal) action.

3. Dispersion according to Claim 1, **characterized in that** the odorant is selected from the group of odorants having action as repellents.

4. Dispersion according to Claim 3, comprising as film-forming polymer a) one or more homopolymers or copolymers comprising one or more monomers from the group consisting of vinyl acetate, vinyl esters of α-branched monocarboxylic acids having 9 to 11 C atoms, vinyl chloride, ethylene, methyl acrylate, methyl methacrylate, ethyl acrylate, ethyl methacrylate, propyl acrylate, propyl methacrylate, n-butyl acrylate, n-butyl methacrylate, 2-ethylhexyl acrylate and styrene.

5. Dispersion according to Claim 3 or 4, comprising as film-forming polymer a) one or more homopolymers or copolymers further comprising 0.1% to 5% by weight, based on the total weight of the polymer, of monomer units deriving from one or more comonomers from the group encompassing ethylenically unsaturated monocarboxylic and dicarboxylic acids, ethylenically unsaturated carboxamides and carbonitriles, monoesters of fumaric acid and maleic acid, and also maleic anhydride, ethylenically unsaturated sulphonic acids and salts thereof.

6. Dispersion according to Claim 3, 4 or 5, comprising CD-odorant complex b) in an amount of 1% to 50%, preferably 5% to 15%, by weight, based in each case on the polymer fraction a).

7. Dispersion according to any of Claims 3 to 6, the pigment fraction and filler fraction being such as to result in a pigment volume concentration PVC of ≥ 10%.

8. Process for preparing the dispersion according to Claim 3 to 7, **characterized in that** the polymer a), in the form of a polymer dispersion or polymer powder, is mixed and homogenized with the other constituents, b) to e), of the formula, in suitable mixers, without addition of non-aqueous volatile solvents.

9. Use of a dispersion according to Claim 1 to 7 as a coating material, in emulsion paints, sealants or polymer renders.

10. Use of a dispersion according to Claim 1 to 7 in emulsion paints, sealants or polymer renders in the exterior sector.

## Revendications

1. Dispersion pouvant être préparée sans solvant, contenant les substances suivantes :
a) au moins un polymère filmogène d'un ou plusieurs monomères choisis dans le groupe comprenant des esters vinyliques, des esters d'acide (méth)acrylique, des composés vinylaromatiques, des oléfines, des 1,3-diènes et des halogénures de vinyle et éventuellement d'autres monomères copolymérisables avec ceux-ci, sous forme de sa dispersion aqueuse de polymère ou poudre de polymère redispersable dans l'eau,
b) un complexe cyclodextrine-substance odorante 1:1 ou 2:1, la cyclodextrine étant choisie dans le groupe constitué par l'α-cyclodextrine, la β-cyclodextrine, la γ-cyclodextrine, la méthyl-β-cyclodextrine, la méthyl-γ-cyclodextrine, l'hydroxypropyl-β-cyclodextrine et l'hydroxypropyl-γ-cyclodextrine,
c) un pigment,
d) une ou plusieurs charges ainsi qu'éventuellement
e) d'autres additifs,
le polymère a) étant mélangé et homogénéisé sous forme d'une dispersion de polymère ou d'une poudre de polymère, dans des mélangeurs convenables, avec les autres composants b) à e) de la composition, sans addition de solvants volatils non aqueux.

2. Dispersion selon la revendication 1, **caractérisée en ce que** la substance odorante est choisie dans le groupe des substances odorantes ayant une action biocide (fongicide, bactéricide, germicide).

3. Dispersion selon la revendication 1, **caractérisée en ce que** la substance odorante est choisie dans le groupe des substances odorantes ayant une action en tant que répulsif.

4. Dispersion selon la revendication 3, qui contient en tant que polymère filmogène a) un ou plusieurs homopolymères ou copolymères contenant un ou plusieurs monomères choisis dans le groupe constitué par l'acétate de vinyle, des esters vinyliques d'acides monocarboxyliques α-ramifiés ayant de 9 à 11 atomes de carbone, le chlorure de vinyle, l'éthylène, l'acrylate de méthyle, le méthacrylate de méthyle, l'acrylate d'éthyle, le méthacrylate d'éthyle, l'acrylate de propyle, le méthacrylate de propyle, l'acrylate de n-butyle, le méthacrylate de n-butyle, l'acrylate de 2-éthylhexyle et le styrène.

5. Dispersion selon la revendication 3 ou 4, qui contient en tant que polymère filmogène a) un ou plusieurs homopolymères ou copolymères qui contiennent encore de 0,1 à 5 % en poids, par rapport au poids total du polymère, de motifs monomères qui dérivent d'un ou plusieurs comonomères choisis dans le groupe comprenant des acides mono- et dicarboxyliques à insaturation éthylénique, des carboxamides et carbonitriles à insaturation éthylénique, des monoesters de l'acide fumarique et de l'acide maléique, ainsi que l'anhydride maléique, des acides sulfoniques à insaturation éthylénique et leurs sels.

6. Dispersion selon la revendication 3, 4 ou 5, qui contient le complexe CD-substance odorante b) en une quantité de 1 à 50 % en poids, de préférence de 5 à 15 % en poids, chaque fois par rapport à la quantité de polymère a).

7. Dispersion selon l'une quelconque des revendications 3 à 6, dans laquelle la quantité de pigment et de charge(s) est choisie de manière qu'il en résulte une concentration en volume de pigment CVP de ≥ 10 %.

8. Procédé pour la préparation de la dispersion selon la revendication 3 à 7, **caractérisé en ce qu'**on mélange et homogénéise le polymère a) sous forme d'une dispersion de polymère ou d'une poudre de polymère, dans des mélangeurs convenables, avec les autres composants b) à e) de la composition, sans addition de solvants volatils non aqueux.

9. Utilisation d'une dispersion selon la revendication 1 à 7, en tant que produit de revêtement, dans des peintures en dispersion, des matières d'étanchéité ou des enduits synthétiques.

10. Utilisation d'une dispersion selon la revendication 1 à 7, dans des peintures en dispersion, des matières d'étanchéité ou des enduits synthétiques pour usage extérieur.
